# EUROPEAN PATENT SPECIFICATION

(11) **EP 1 760 661 B1**
(45) Date of publication and mention of the grant of the patent: **06.06.2018**
(21) Application number: 06254473.9
(22) Date of filing: 29.08.2006
(51) Int. Cl.: G06T 7/00, A61B 5/00

(54) **Segmentation and registration of multimodal images using physiological data**
Segmentierung und Registrierung multimodaler Bilder unter Verwendung physiologischer Daten
Segmentation et recalage d'images multimodales à l'aide de données physiologiques

(30) Priority: 30.08.2005 US 215435
(43) Date of publication of application: 07.03.2007
(73) Proprietor: Biosense Webster, Inc., Diamond Bar, CA 91765 (US)
(72) Inventor: Preiss, Assaf, Shimshit 17906 (IL); Schwartz, Yitzhack, Haifa 34606 (IL)
(74) Representative: Tunstall, Christopher Stephen

(56) References cited:
- US-A- 5 738 096
- US-A1- 2003 013 958
- REDDY V Y ET AL: "Integration of cardiac magnetic resonance imaging with three-dimensional electroanatomic mapping to guide left ventricular catheter manipulation - Feasibility in a porcine model of healed myocardial infarction" JOURNAL OF THE AMERICAN COLLEGE OF CARDIOLOGY, ELSEVIER, NEW YORK, NY, US, vol. 44, no. 11, 7 December 2004 (2004-12-07), pages 2202-2213, XP004669554 ISSN: 0735-1097
- TOPS ET AL: "Fusion of multislice computed tomography imaging with three-dimensional electroanatomic mapping to guide radiofrequency catheter ablation procedures" HEART RHYTHM, ELSEVIER, vol. 2, no. 10, 1 October 2005 (2005-10-01), pages 1076-1081, XP005091441 ISSN: 1547-5271
- GEPSTEIN L ET AL: "ELECTROANATOMICAL MAPPING OF THE HEART: BASIC CONCEPTS AND IMPLICATIONS FOR THE TREATMENT OF CARDIAC ARRHYTHMIAS" PACE - PACING AND CLINICAL ELECTROPHYSIOLOGY, BLACWELL FUTURA PUBLISHING, MALDEN, MA, US, vol. 21, no. 6, 1 June 1998 (1998-06-01), pages 1268-1278, XP000772441 ISSN: 0147-8389
- BOLOTIN G ET AL: "Three-dimensional electromechanical mapping: imaging in the operating room of the future." THE ANNALS OF THORACIC SURGERY SEP 2001, vol. 72, no. 3, September 2001 (2001-09), pages S1083-S1089, XP002523502 ISSN: 0003-4975
- SIMON R D B ET AL: "Electroanatomic Mapping of the Right Atrium with a Right Atrial Basket Catheter and Three-Dimensional Inracardiac Echocardiography" PACE - PACING AND CLINICAL ELECTROPHYSIOLOGY, BLACWELL FUTURA PUBLISHING, MALDEN, MA, US, vol. 27, no. 3, 1 March 2004 (2004-03-01), pages 318-326, XP008083825 ISSN: 0147-8389
- GHANEM R N ET AL: "Heart-surface reconstruction and ecg electrodes localization using fluoroscopy, epipolar geometry and stereovision: application to noninvasive imaging of cardiac electrical activity" IEEE TRANSACTIONS ON MEDICAL IMAGING, IEEE SERVICE CENTER, PISCATAWAY, NJ, US, vol. 22, no. 10, 1 October 2003 (2003-10-01), pages 1307-1318, XP011101829 ISSN: 0278-0062
- LILIANA IRONI ET AL: "Electrocardiographic Imaging: Towards Automated Interpretation of Activation Maps" ARTIFICIAL INTELLIGENCE IN MEDICINE LECTURE NOTES IN COMPUTER SCIENCE;LECTURE NOTES IN ARTIFICIAL INTELLIG ENCE;LNCS, SPRINGER, BERLIN, DE, vol. 3581, 1 January 2005 (2005-01-01), pages 323-332, XP019016144 ISBN: 978-3-540-27831-3

## Description

### BACKGROUND OF THE INVENTION

### Field of the Invention

This invention relates to anatomic imaging and electro-anatomical mapping. More particularly, this invention relates to synchronized display of images and electro-anatomical maps of the heart that are acquired by different modalities.

### Description of the Related Art

Methods for three-dimensional geometrical mapping and reconstruction of the endocardial surface are known in the art. For example, U.S. Patent No. 5,738,096 describes methods for mapping the endocardium based on bringing a probe into contact with multiple locations on a wall of the heart, and determining position coordinates of the probe at each of the locations. The position coordinates are combined to form a map of at least a portion of the heart.

Hybrid catheters are now known that perform ultrasound imaging in conjunction with position sensing. Such devices are disclosed, for example, in commonly assigned U.S. Patent Nos. 6,690,963, 6,716,166 and 6,773 402. Medical applications include three-dimensional mapping of a cavity of the body, as well as measurement of chamber wall thickness and wall velocity and mapping of electrical activity. In medical applications, it is common to acquire maps and images of body organs by different modalities, which are to be interpreted in relationship to one another. An example is correlation of an electro-anatomical map of the heart and an image, such as a three-dimensional ultrasound image.

Commercial electrophysiological and physical mapping systems based on detecting the position of a probe inside the body are presently available. Among them, the Carto-Biosense® system, available from Biosense Webster Inc., 3333 Diamond Canyon Road Diamond Bar, CA 91765, is a system for automatic association and mapping of local electrical activity with catheter location.

Existing methods for registering anatomical images and electro-anatomical maps with three-dimensional images acquired by a different modality generally rely on location data. The mapping catheter is placed at a number of known locations in the organ of interest, such as the heart, and the position coordinates are recorded. These same locations are marked or otherwise recorded in the three-dimensional image. This technique generally requires the operator of the system to take time to find and mark the desired locations for the purpose of registration, in addition to the actions taken as part of the mapping procedure itself.

U.S. Patent No. 5,568,384, issued to Robb, et al.*,* describes a method for synthesizing three-dimensional multimodality image sets into a single composite image with accurate registration and congruence. Surfaces are initially extracted from two or more different images to be matched using semi-automatic segmentation techniques. These surfaces are represented as contours with common features to be matched. A distance transformation is performed for one surface image, and a cost function for the matching process is developed using the distance image. The geometric transformation includes three-dimensional translation, rotation and scaling to accommodate images of different position, orientation and size. The matching process involves efficiently searching this multi-parameter space and adjusting a surface or surfaces to find the best fit among them, which minimizes the cost function. The local minima problem is addressed by using a large number of starting points. A pyramid multi-resolution approach is employed to speed up both the distance transformation computation and the multi-parameter minimization processes. Robustness in noise handling is accomplished using multiple thresholds embedded in the multi-resolution search. The method can register both partially overlapped and fragmented surfaces.

In the document, A Review of Cardiac Image Registration Methods, Timo Mäkelä, et al., IEEE Transactions on Medical Imaging, Vol.21, No. 9, p. 1011, September 2002, the current status of cardiac image registration methods is reviewed. Registration of cardiac images is noted to be a particularly complex problem for image registration because the heart is a non-rigid moving organ inside a moving body, and has relatively few accurately localized anatomical landmarks.

### SUMMARY OF THE INVENTION

According to disclosed embodiments of the invention, alternative systems and methods are provided for registering maps with images, including segmentation of three-dimensional images and registration of such images with an anatomical map using physiological or functional information in the map, combined with specific location points. In a clinical context, physicians often mentally integrate image information from different modalities. Registration, based on computer programs using physiological data according to the invention, offers better accuracy and is more rapid.

In one embodiment of the invention, voltage values in an electro-anatomical map are identified with features in a preacquired or real-time three-dimensional image that are known to generate such values. For example, scar tissue in the heart typically exhibits lower voltage than healthy tissue in an electro-anatomical map. A scar that is outlined as a low voltage area on an electro-anatomical map may be registered with a corresponding structure that is delineated in a three-dimensional image.
trodes will appear in the CT image. ECG measurements that are performed using the electrodes provide an electrical model that can be projected inward to the heart surface. Electro-anatomical maps of the heart likewise produce an electrical model of the heart that can be projected outward to the body surface. The two electrical models may be registered with one another in order to register the electro-anatomical map of the heart with the CT image.

The invention provides a method for mapping a structure in a body of a subject, which is carried out by capturing a three-dimensional image of the structure, generating a three-dimensional map of the structure having functional information relating to the structure measured at multiple points, registering the image with the map by automatically identifying at least one of the functional features on the map with at least a corresponding one of the anatomical features in the image, and displaying the functional information from the map in registration with the image.

One aspect of the method includes inserting a probe into the structure, including a position sensor for determining position and orientation information of the probe.

In another aspect of the method, generating a functional model comprises generating an electrical model by contacting the probe with multiple contact points on the structure, and using the position sensor of the probe to obtain position and orientation information associated with each of the contact points.

According to another aspect of the method, the structure includes a heart, and wherein the functional information includes a feature of a local electrocardiogram taken at each of the contact points.

According to one aspect of the method, the functional information includes magnitudes of electrical voltages at the contact points.

A further aspect of the method includes identifying a myocardial scar in a heart by delineating an area of the heart, wherein the contact points in the area have lower voltages than the contact points that are located outside the area.

An additional aspect of the method includes identifying a valve of the heart by delineating an area of the heart, wherein the contact points have voltages that differ from voltages of the contact points that are located outside the area.

According to a further aspect of the method, the functional information includes impedances between a surface of the body and respective ones of the contact points.

According to still another aspect of the method, the image is a computed tomographic image of a thorax of the body that includes a representation of a heart thereof.

An additional aspect of the method includes placing a plurality of surface electrodes on the thorax of the subject, and generating an external electrical model by performing an electrocardiogram using the surface electrodes, wherein registering the image and the map comprises the additional steps of projecting the electrical model outwardly onto the representation of the heart and projecting the external electrical model inwardly onto the representation of the heart to place the external electrical model in registration with the electrical model and with the representation of the heart.

According to yet another aspect of the method, the image is an ultrasound image.

The invention provides an apparatus for mapping a structure in a body of a subject, including an imaging device for capturing a three-dimensional image of the structure, and a processor linked to the imaging device, wherein the processor is operative for generating a three-dimensional functional map of the structure containing functional information relating to the structure measured at multiple points on the structure. The processor is operative for registering the image with the map by automatically identifying at least one of the functional features on the map with a corresponding one of the anatomical features in the image. The apparatus includes a display device linked to the processor for displaying the functional information from the map in registration with the image.

### BRIEF DESCRIPTION OF THE DRAWINGS

For a better understanding of the present invention, reference is made to the detailed description of the invention, by way of example, which is to be read in conjunction with the following drawings, wherein like elements are given like reference numerals, and wherein:
Fig. 1 is an illustration of a system for imaging and mapping a heart of a patient in accordance with an embodiment of the invention;
Fig. 2 schematically illustrates an embodiment of the distal end of s catheter used in the system shown in Fig. 1, in accordance with an embodiment of the invention;
Fig. 3 is a simplified geometric representation of an image of the heart, which has been prepared for registration with another diagnostic image positioned in accordance with a disclosed embodiment of the invention;
Fig. 4 is a schematic exploded view of a diagnostic image of the heart, in accordance with a disclosed embodiment of the invention;
Fig. 5 is a simplified representation of an electro-anatomical map of a heart, a corresponding three-dimensional anatomic image, and a composite image in which a portion of the electro-anatomical map is shown in registration with the anatomic image, in accordance with a disclosed embodiment of the invention; and
Fig. 6 is a simplified representation of an electro-anatomical map of a heart, a corresponding three-dimensional anatomic image, and a composite image in which a portion of the electro-anatomical map is shwon in registration with the anatomic image, in accordance with an alternate embodiment of the invention.

### DETAILED DESCRIPTION OF THE INVENTION

In the following description, numerous specific details are set forth in order to provide a thorough understanding of the present invention. It will be apparent to one skilled in the art, however, that the present invention may be practiced without these specific details. In other instances, well-known circuits, control logic, and the details of computer program instructions for conventional algorithms and processes have not been shown in detail in order not to obscure the present invention unnecessarily.

Software programming code, which embodies aspects of the present invention, is typically maintained in permanent storage, such as a computer readable medium. In a client-server environment, such software programming code may be stored on a client or a server. The software programming code may be embodied on any of a variety of known media for use with a data processing system. This includes, but is not limited to, magnetic and optical storage devices such as disk drives, magnetic tape, compact discs (CD's), digital video discs (DVD's), and computer instruction signals embodied in a transmission medium with or without a carrier wave upon which the signals are modulated. For example, the transmission medium may include a communications network, such as the Internet. In addition, while the invention may be embodied in computer software, the functions necessary to implement the invention may alternatively be embodied in part or in whole using hardware components such as application-specific integrated circuits or other hardware, or some combination of hardware components and software.

### System Overview

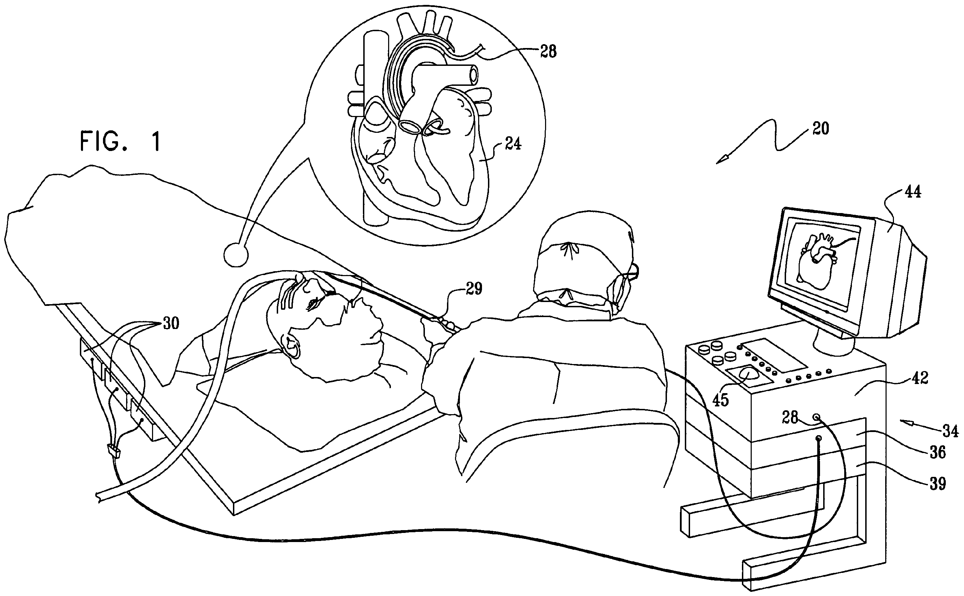

Turning now to the drawings, reference is initially made to Fig. 1, which is an illustration of a system 20 for imaging and mapping a heart 24 of a patient, and which is suitable for performing diagnostic or therapeutic procedures involving the heart 24, in accordance with an embodiment of the present invention. The system comprises a catheter 28, which is percutaneously inserted by a physician into a chamber or vascular structure of the heart. The catheter 28 typically comprises a handle 29 for operation of the catheter by the physician. Suitable controls on the handle enable the physician to steer, position and orient the distal end of the catheter as desired.

The system 20 comprises a positioning subsystem that measures location and orientation coordinates of the catheter 28. Throughout this patent application, the term "location" refers to the spatial coordinates of the catheter, and the term "orientation" refers to its angular coordinates or rotation. The term "position" refers to the full positional information of the catheter, comprising both location and orientation coordinates.

In one embodiment, the positioning subsystem comprises a magnetic position tracking system that determines the position and orientation of the catheter 28. The positioning subsystem generates magnetic fields in a predefined working volume its vicinity and senses these fields at the catheter. The positioning subsystem typically comprises a set of external radiators, such as field generating coils 30, which are located in fixed, known positions external to the patient. The coils 30 generate fields, typically electromagnetic fields, in the vicinity of the heart 24.

In an alternative embodiment, a radiator in the catheter, such as a coil, generates electromagnetic fields, which are received by sensors (not shown) outside the patient's body.

The position sensor transmits, in response to the sensed fields, position-related electrical signals over cables 33 running through the catheter to a console 34. Alternatively, the position sensor may transmit signals to the console over a wireless link. The console comprises a positioning processor 36 that calculates the location and orientation of the catheter 28 based on the signals sent by a position sensor 32. The positioning processor 36 typically receives, amplifies, filters, digitizes, and otherwise processes signals from the catheter 28.

Some position tracking systems that may be used for this purpose are described, for example, in U.S. Patents 6,690,963, 6,618,612 and 6,332,089, and U.S. Patent Application Publications 2002/0065455 A1, 2004/0147920 A1, and 2004/0068178 A1. Although the positioning subsystem shown in Fig. 1 uses magnetic fields, the methods described below may be implemented using any other suitable positioning subsystem, such as systems based on electromagnetic fields, acoustic or ultrasonic measurements.

Alternatively, the system 20 can be realized as the Carto-Biosense® Navigation System, available from Biosense Webster, Inc., 3333 Diamond Canyon Road, Diamond Bar, CA 91765, suitably modified to execute the procedures described hereinbelow. For example, the system 20 may be adapted, *mutatis mutandis,* to employ the catheters disclosed in the above-noted U.S. Patent Nos. 6,716,166 and 6,773,402 in order to acquire ultrasound images for display in near realtime ultrasound images concurrently with an image or representation of the position of a deployment catheter in the same or different sessions, and in many different combinations.

When used for inserting therapy devices and implants, the catheter 28 is provided with a flexible guide wire, which is fed into a desired site. Accessory ports, such as a side port (not shown) may optionally be provided to accommodate the requirements for deploying implants and therapy devices.

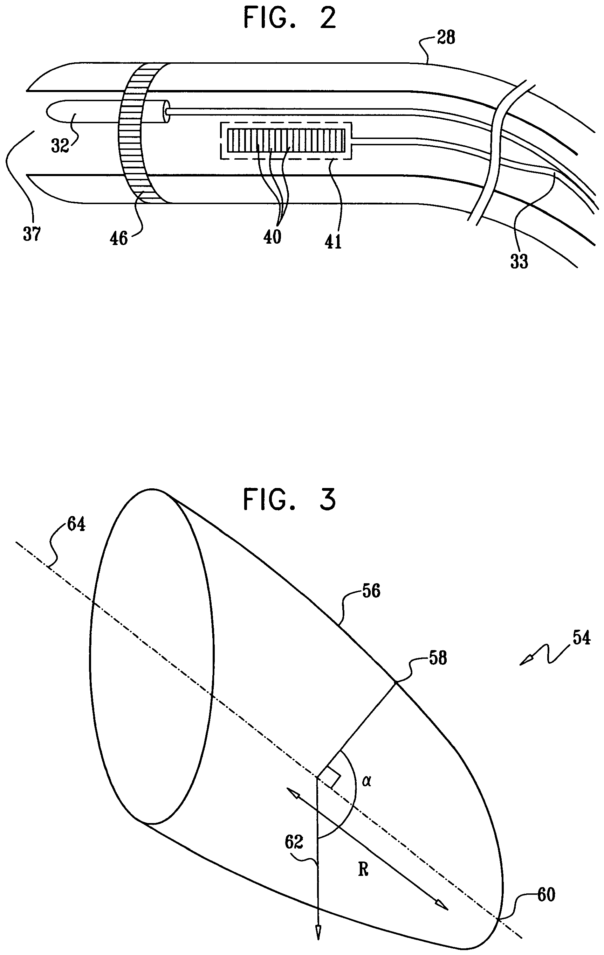

Reference is now made to Fig. 2, which schematically illustrates an embodiment of the distal end of the catheter 28 (Fig. 1), in accordance with an embodiment of the present invention. The fields generated by the field generating coils 30 (Fig. 1) are sensed by the position sensor 32 inside the catheter 28. The catheter 28 comprises an ultrasonic imaging sensor. The ultrasonic sensor typically comprises an array of ultrasonic transducers 40. In one embodiment, the transducers are piezo-electric transducers. The ultrasonic transducers are positioned in or adjacent to a window 41, which defines an opening within the body or wall of the catheter. The catheter 28 typically has at least one lumen 37, which can admit a guide wire and guide tube to aid in deployment of a therapeutic device.

The transducers 40 operate as a phased array, jointly transmitting an ultrasound beam from the array aperture through the window 23. Although the transducers are shown arranged in a linear array configuration, other array configurations can be used, such as circular or convex configurations. In one embodiment, the array transmits a short burst of ultrasound energy and then switches to a receiving mode for receiving the ultrasound signals reflected from the surrounding tissue. Typically, the transducers 40 are driven individually in a controlled manner in order to steer the ultrasound beam in a desired direction. By appropriate timing of the transducers, the produced ultrasound beam can be given a concentrically curved wave front, to focus the beam at a given distance from the transducer array. Thus, the system 20 (Fig. 1) uses the transducer array as a phased array and implements a transmit/receive scanning mechanism that enables the steering and focusing of the ultrasound beam, so as to produce two-dimensional ultrasound images.

In one embodiment, the ultrasonic sensor comprises between sixteen and sixty-four transducers 40, preferably between forty-eight and sixty-four transducers. Typically, the transducers generate the ultrasound energy at a center frequency in the range of 5-10 MHz, with a typical penetration depth of 14 cm. The penetration depth typically ranges from several millimeters to around 16 centimeters, and depends upon the ultrasonic sensor characteristics, the characteristics of the surrounding tissue and the operating frequency. In alternative embodiments, other suitable frequency ranges and penetration depths can be used.

After receiving the reflected ultrasound echoes, electric signals based on the reflected acoustic signals or echoes are sent by transducers 40 over cables 33 through the catheter 28 to an image processor 42 (Fig. 1) in the console 34, which transforms them into two-dimensional, typically sector-shaped ultrasound images. The image processor 42 typically computes or determines position and orientation information, displays real-time ultrasound images, performs three-dimensional image or volume reconstructions and other functions, which will all be described in greater detail below.

In some embodiments, the image processor uses the ultrasound images and the positional information to produce a three-dimensional model of a target structure of the patient's heart. The three-dimensional model is presented to the physician as a two-dimensional projection on a display 44.

In some embodiments, the distal end of the catheter also comprises at least one electrode 46 for performing diagnostic functions, therapeutic functions or both, such as electro-physiological mapping and radio frequency (RF) ablation. In one embodiment, the electrode 46 is used for sensing local electrical potentials. The electrical potentials measured by the electrode 46 may be used in mapping the local electrical activity at contact points of the endocardial surface. When the electrode 46 is brought into contact or proximity with a point on the inner surface of the heart 24 (Fig. 1), it measures the local electrical potential at that point. The measured potentials are converted into electrical signals and sent through the catheter to the image processor for display as a map reflecting the functional data or activity at each contact point. In other embodiments, the local electrical potentials are obtained from another catheter comprising suitable electrodes and a position sensor, all connected to the console 34. In some applications, the electrode 46 can be used to determine when the catheter is in contact with a valve, since the electrical potentials are weaker there than in the myocardium.

Although the electrode 46 is shown as being a single ring electrode, the catheter may comprise any number of electrodes in any form. For example, the catheter may comprise two or more ring electrodes, a plurality or array of point electrodes, a tip electrode, or any combination of these types of electrodes for performing the diagnostic and therapeutic functions outlined above.

The position sensor 32 is typically located within the distal end of the catheter 28, adjacent to the electrode 46 and the transducers 40. Typically, the mutual positional and orientational offsets between the position sensor 32, electrode 46 and transducers 40 of the ultrasonic sensor are constant. These offsets are typically used by the positioning processor 36 to derive the coordinates of the ultrasonic sensor and of the electrode 46, given the measured position of the position sensor 32. In another embodiment, the catheter 28 comprises two or more position sensors 32, each having constant positional and orientational offsets with respect to the electrode 46 and the transducers 40. In some embodiments, the offsets (or equivalent calibration parameters) are pre-calibrated and stored in the positioning processor 36. Alternatively, the offsets can be stored in a memory device (such as an electrically programmable read-only memory, or EPROM) fitted into the handle 29 (Fig. 1) of the catheter 28.

The position sensor 32 typically comprises three non-concentric coils (not shown), such as described in U.S. Patent No. 6,690,963, cited above. Alternatively, any other suitable position sensor arrangement can be used, such as sensors comprising any number of concentric or non-concentric coils, Hall-effect sensors or magneto-resistive sensors.

Typically, both the ultrasound images and the position measurements are synchronized with the heart cycle, by gating signal and image capture relative to a body-surface electrocardiogram (ECG) signal or intra-cardiac electrocardiogram. (In one embodiment, the ECG signal can be produced by the electrode 46.) Since features of the heart change their shape and position during the heart's periodic contraction and relaxation, the entire imaging process is typically performed at a particular timing with respect to this period. In some embodiments, additional measurements taken by the catheter, such as measurements of various tissue characteristics, temperature and blood flow measurements, are also synchronized to the electrocardiogram (ECG) signal. These measurements are also associated with corresponding position measurements taken by the position sensor 32. The additional measurements are typically overlaid on the reconstructed three-dimensional model, as will be explained below.

In some embodiments, the position measurements and the acquisition of the ultrasound images are synchronized to an internally generated signal produced by the system 20. For example, the synchronization mechanism can be used to avoid interference in the ultrasound images caused by a certain signal. In this example, the timing of image acquisition and position measurement is set to a particular offset with respect to the interfering signal, so that images are acquired without interference. The offset can be adjusted occasionally to maintain interference-free image acquisition. Alternatively, the measurement and acquisition can be synchronized to an externally supplied synchronization signal.

In one embodiment, the system 20 comprises an ultrasound driver 39 that drives the ultrasound transducers 40. One example of a suitable ultrasound driver, which can be used for this purpose is an AN2300™ ultrasound system produced by Analogic Corporation, 8 Centennial Drive, Peabody, MA 01960. In this embodiment, the ultrasound driver performs some of the functions of the image processor 42, driving the ultrasonic sensor and producing the two-dimensional ultrasound images. The ultrasound driver may support different imaging modes such as B-mode, M-mode, CW Doppler and color flow Doppler, as are known in the art.

Typically, the positioning and image processors are implemented using a general-purpose computer, which is programmed in software to carry out the functions described herein. The software may be downloaded to the computer in electronic form, over a network, for example, or it may alternatively be supplied to the computer on tangible media, such as CD-ROM. The positioning processor and image processor may be implemented using separate computers or using a single computer, or may be integrated with other computing functions of the system 20. Additionally or alternatively, at least some of the positioning and image processing functions may be performed using dedicated hardware.

### Two-dimensional Anatomic Imaging

Referring again to Fig. 1, gated images of the heart are created, e.g., ultrasound, SPECT, images and correlated with location data of the catheter 28. The gated images can be registered with another image, or with the position of the same or a different catheter used for deployment of a therapeutic device in the coronary sinus. Suitable registration techniques are disclosed in U.S. Patent No. 6,650,927, of common assignee herewith. The technique is briefly described:

Reference is now made to Fig. 3, which is a simplified geometric representation of an image 54 of the heart, which has been prepared for registration with another diagnostic image in accordance with a disclosed embodiment of the invention. Details of the preparation of the image 54 are described in further detail hereinbelow. A surface 56 corresponds approximately to the surface of the heart. A coordinate system is defined, in which each point 58 on the surface 56 is represented by a distance R from an apex 60 and an angle *a* relative to a downward direction 62 (i.e., ventrally and caudad relative to the subject 26 (Fig. 1). In order to register another structure with the image 54, an axis 64 and the apex 60 are identified on the image 54 and aligned with corresponding positions, landmarks or fiducial marks of the structure to be registered, using location information provided by the sensors on the catheter 28 (Fig. 1). This is preferably automatic, but additionally or alternatively can be done or assisted by an operator. The scale of the structure to be registered is adjusted so that its dimensions match that of the image 54 as closely as possible.

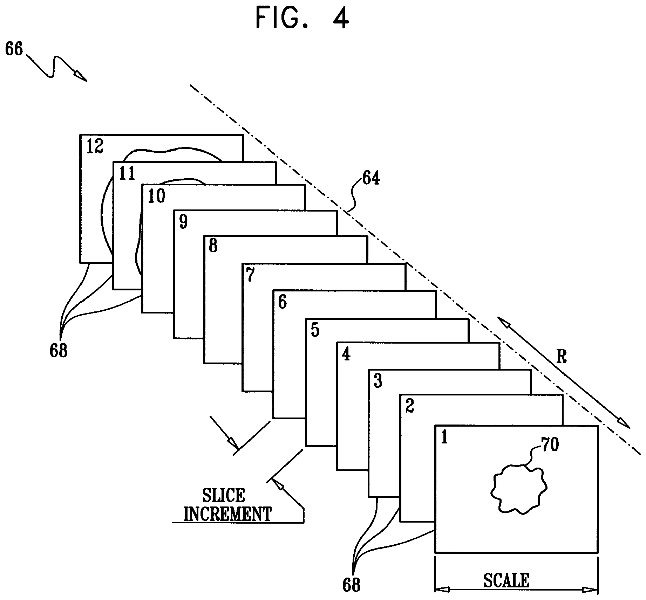

Reference is now made to Fig. 4, which is a schematic exploded view of a diagnostic image 66 of the heart 24 (Fig. 1), in accordance with a disclosed embodiment of the invention. The view is generated using a bullseye rendition technique. The image 66 comprises a stack of parallel slices 68, which are perpendicular to the axis 64. The slices are typically taken at a fixed slice increment along the axis 64. Each slice shows a section 70.

### Three-dimensional Anatomic Imaging

Referring again to Fig. 1, three-dimensional imaging is described in commonly assigned Application No. 11/115,002 filed on April 26, entitled *Three-Dimensional Cardiac Imaging Using Ultrasound Contour* Reconstruction. A brief description of the method will facilitate understanding of the present invention.

Essentially, the disclosed method combines multiple two-dimensional ultrasound images, acquired at different positions of the catheter 28 as described above, into a single three-dimensional model of the target structure. Typically, the physician inserts the catheter 28 through a suitable blood vessel into a chamber of the heart, and then scans the target structure by moving the catheter between different positions inside the chamber. In each catheter position, the image processor 42 acquires and produces a two-dimensional ultrasound image,

Referring again to Fig. 1, during deployment of a therapeutic device or implant, the positioning subsystem of the system 20 measures and calculates the current position of the catheter 28. The calculated position is stored together with the corresponding slice or slices 68 (Fig. 3). Typically, each position of the catheter 28 is represented in coordinate form, such as a six-dimensional coordinate (X, Y, Z axis positions, and pitch, yaw and roll angular orientations).

The image processor 42 subsequently assigns three-dimensional coordinates to the contours of interest, identified in the set of images. The location and orientation of the planes of these images in three-dimensional space are known by virtue of the positional information, stored together with the images. Therefore, the image processor is able to determine the three-dimensional coordinates of any pixel in the two-dimensional images. When assigning the coordinates, the image processor typically uses stored calibration data comprising position and orientation offsets between the position sensor and the ultrasonic sensor, as described above.

Alternatively, the system 20 (Fig. 1) can be used for three-dimensional display and projection of two-dimensional ultrasound images, without reconstructing a three-dimensional model. For example, the physician can acquire a single two-dimensional ultrasound image. Contours of interest on this image can be tagged using the procedures described below. The system 20 can then orient and project the ultrasound image in three-dimensional space. During a medical procedure the system can continuously track and display the three-dimensional position of the catheter performing the medical procedure, which may be different from the catheter that acquired the image onto which the catheter now performing the medical procedure is being registered.

### Functional Imaging Techniques

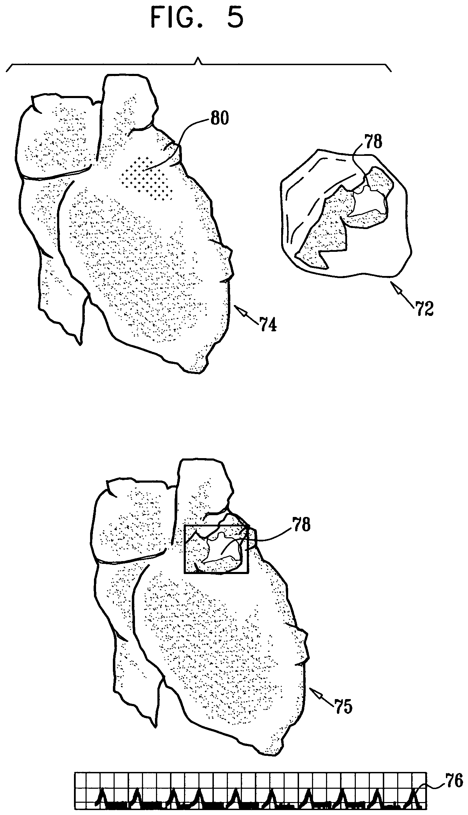

Reference is now made to Fig. 5, which shows an electro-anatomical map 72 of a heart, a corresponding three-dimensional anatomic image 74, and a composite image 75, in which a replicated portion of a portion of the electro-anatomical map 72 is in registration with the anatomic image 74, in accordance with a disclosed embodiment of the invention. The images are acquired and reconstructed as described above. A reference ECG tracing 76 is shown in the lower portion of the figure. The electro-anatomical map 72 discloses an area 78 of relatively low voltage. An area of interest 80 is delineated on the anatomic image 74, which is consistent with a myocardial scar. Scar tissue in the heart affects myocardial function in that it typically exhibits lower voltage than healthy tissue in an electro-anatomical map, as indicated by the area 78. The composite image is formed by registering the area 80 with the area 78.

### Alternate Embodiments

In another embodiment of the invention, electrical potentials may be used for segmentation of an image. For example, the locations and shapes of valves in the heart may be delineated on the basis of differences in electrical potentials between the valves and the surrounding endocardium.

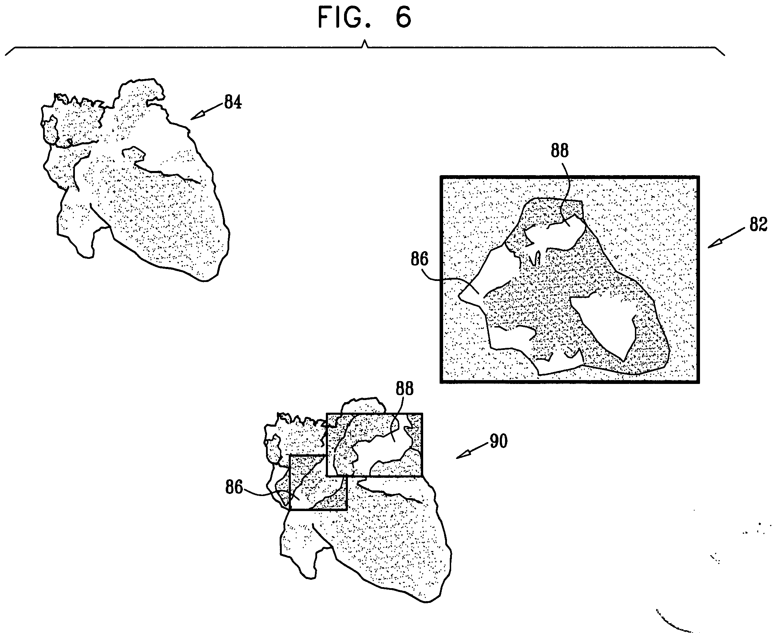

Reference is now made to Fig. 6, which shows an electro-anatomical map 82 of a heart, a corresponding concurrently acquired three-dimensional anatomic image 84, and a composite image 90 in which a replicated portion of the electro-anatomical map 82 is in registration with the anatomic image 84, in accordance with a disclosed embodiment of the invention. On the anatomic image 84 the locations of the mitral valve and aortic valve can be determined by the operator, based on the morphologic appearance of the heart. On the electro-anatomical map 82, areas 86, 88 of relatively low electrical activity indicate the aortic and mitral valves, respectively. After relevant portions of the electro-anatomical map 82 including the areas 86, 88, are in registration with the operator-identified areas of the anatomic image 84, the resulting composite image 90 of the entire heart becomes available to the operator in near realtime.

Other electrical features may also be used in segmentation and registration. For example, movement of a mapping catheter from the atrium to the ventricle may be identified by disappearance of the P-wave in the local electrocardiogram as the catheter enters the ventricle. As another example, in impedance-based location systems, in which electrical impedance between a mapping catheter and a body surface electrode is measured, the location of the pulmonary veins may be identified by a rise in impedance as the catheter moves from the left atrium into the veins.

In one embodiment of the invention, NOGA™ software, available from Biosense-Webster, is employed for registration. The software employs a filter to detect the P-wave in a bipolar EKG, and thus can distinguish points that are on the fibrous ring of the valves at the basal zone from points that are clearly in the atrium. The algorithm used essentially defines the body surface QRS complex, and its P-wave location, and then looks for a deflection in that time range in the bipolar window. Two predefined parameters must be met: (1) The peak-to-peak voltage of the deflection must be in the range of 0 to 0.5mV. (2) The ratio of the deflection to the magnitude of the QRS complex must be in the range of 0 to 100%. In general, it is considered that a peak-to-peak voltage of 0.1mV already exceeds the noise level and represents a true deflection signal. A ratio of 25% seems to be sufficient. If the first or the second parameters are increased, fewer points will meet the criteria and more truly basal points will be missed. On the other hand, decreasing the parameters result in deletion of valid points (increased false positive). In a typical left ventricular map the algorithm typically detects 3-10 points, which are almost always indeed basal locations.

In yet another embodiment of the invention, if a patient wears a "vest" of body surface electrodes during computed tomographic (CT) imaging of the thorax, the electrodes will appear in the CT image. ECG measurements that are performed using the electrodes provide an electrical model that can be projected inward to the heart surface. Electro-anatomical maps of the heart likewise produce an electrical model of the heart that can be projected outward to the body surface. The two electrical models may be registered with one another in order to register the electro-anatomical map of the heart with the CT image. The registration algorithm uses both location and electrical activity information.

Other physiological data that may be mapped and used in image registration and segmentation include temperature, blood flow rate, chemical properties and mechanical activity. For example, areas of high-speed flow detected by an ultrasound catheters, as disclosed, e.g., in the above-noted U.S. Patent Nos. 6,716,166 and 6,773,402 in a Doppler image may be identified and registered with stenoses in blood vessels observed in a three-dimensional anatomic image. As another example, a chemical sensor may be used to identify areas of the heart with low NADPH levels, indicative of ischemia. Such areas may be registered with corresponding ischemic areas observed on images obtained using magnetic resonance spectroscopy. The technique described in the article Quantitative Measurements of Cardiac Phosphorus Metabolites in Coronary Artery Disease by 31P Magnetic Resonance Spectroscopy, Takahiro Yabe et al., Circulation. 1995;92:15-23 is suitable for displaying such areas.

It will be appreciated by persons skilled in the art that the present invention is not limited to what has been particularly shown and described hereinabove. Rather, the scope of the present invention includes both combinations and sub-combinations of the various features described hereinabove, as well as variations and modifications thereof that are not in the prior art, which would occur to persons skilled in the art upon reading the foregoing description.

## Claims

1. An apparatus for mapping a structure in a body of a subject, said structure comprising a heart, the apparatus comprising:
an imaging device for capturing a three-dimensional image of said structure, said structure having anatomical features that appear in said image;
a probe linked to said processor and adapted for insertion into said structure, said probe having a position sensor for determining position and orientation information of said probe;
a processor linked to said imaging device, said processor being operative for generating a functional model comprising a three-dimensional map of said structure comprising functional information relating to said structure measured at multiple points on said structure, said map exhibiting functional features of said structure,
wherein said functional model comprises an electrical model when said probe is contacted with multiple contact points on said structure, and responsively to said position sensor of said probe said processor is operative to obtain position and orientation information associated with each of said contact points and wherein said structure comprises a heart, and wherein said functional information comprises a feature of a local electrocardiogram taken at each of said contact points; and
a display device linked to said processor for displaying said functional information from said map in registration with said image
**characterised in that** said processor is operative for registering said image with said map by automatically identifying at least one of said functional features with at least a corresponding one of said anatomical features in said image by using voltage values in said map to identify one of said functional features with a corresponding one of said anatomical features in the three-dimensional image that are known to generate such values.

2. The apparatus according to claim 1, wherein said functional information comprises magnitudes of electrical voltages at said contact points.

3. The apparatus according to claim 1, further wherein said functional information comprises impedances between a surface of said body and respective ones of said contact points.

4. The apparatus according to claim 1herein said image is a computed tomographic image of a thorax of said body that includes a representation of a heart thereof.

5. The apparatus according to claim 1, wherein said image is an ultrasound image.

## Patentansprüche

1. Vorrichtung zur Kartierung einer Struktur in einem Körper eines Subjekts, wobei die Struktur ein Herz umfasst, die Vorrichtung umfassend:
eine Bildgebungsvorrichtung zum Erfassen eines dreidimensionalen Bildes der Struktur, wobei die Struktur anatomische Merkmale hat, die in dem Bild erscheinen;
eine Sonde, die mit dem Prozessor verbunden und zum Einsetzen in die Struktur geeignet ist, wobei die Sonde einen Positionssensor zum Ermitteln von Positions- und Orientierungsinformationen der Sonde hat;
einen Prozessor, der mit der Bildgebungsvorrichtung verbunden ist, wobei der Prozessor betriebsbereit ist, ein Funktionsmodell zu generieren, das eine dreidimensionale Karte der Struktur umfasst, die Funktionsinformationen bezüglich der Struktur umfasst, die an mehreren Punkten an der Struktur gemessen werden, wobei die Karte Funktionsmerkmale der Struktur zeigt,
wobei das Funktionsmodell ein elektrisches Modell umfasst, wenn die Sonde mit mehreren Kontaktpunkten auf der Struktur in Kontakt gebracht wird, und der Prozessor, auf den Positionssensor der Sonde ansprechend, betriebsbereit ist, Positions- und Orientierungsinformationen zu erlangen, die mit jedem der Kontaktpunkte verknüpft sind, und wobei die Struktur ein Herz umfasst und wobei die Funktionsinformationen ein Merkmal eines lokalen Elektrokardiogramms umfassen, das an jedem der Kontaktpunkte erstellt wird; und
eine Anzeigevorrichtung, die mit dem Prozessor verbunden ist, zur Anzeige der Funktionsinformationen aus der Karte in Registrierung mit dem Bild,
**dadurch gekennzeichnet, dass** der Prozessor betriebsbereit ist, das Bild mit der Karte, durch automatisches Identifizieren zumindest eines der Funktionsmerkmale mit zumindest einem entsprechenden der anatomischen Merkmale in dem Bild unter Verwendung von Spannungswerten in der Karte zu registrieren, um eines der Funktionsmerkmale mit einem entsprechenden der anatomischen Merkmale in dem dreidimensionalen Bild zu identifizieren, von welchen bekannt ist, dass sie solche Werte generieren.

2. Vorrichtung nach Anspruch 1, wobei die Funktionsinformationen Größenordnungen von elektrischen Spannungen an den Kontaktpunkten umfassen.

3. Vorrichtung nach Anspruch 1, wobei ferner die Funktionsinformationen Scheinwiderstände zwischen einer Oberfläche des Körpers und entsprechenden der Kontaktpunkte umfassen.

4. Vorrichtung nach Anspruch 1, wobei das Bild ein Computertomographiebild eines Thorax des Körpers ist, das eine Darstellung eines Herzens in diesem enthält.

5. Vorrichtung nach Anspruch 1, wobei das Bild ein Ultraschallbild ist.

## Revendications

1. Appareil pour cartographier une structure dans un corps d'un sujet, ladite structure comprenant un coeur, l'appareil comprenant :
un dispositif d'imagerie pour capturer une image tridimensionnelle de ladite structure, ladite structure ayant des caractéristiques anatomiques qui apparaissent dans ladite image ;
une sonde reliée audit processeur et adaptée pour être insérée dans ladite structure, ladite sonde ayant un capteur de position pour déterminer des informations de position et d'orientation de ladite sonde ;
un processeur relié audit dispositif d'imagerie, ledit processeur étant opérationnel pour générer un modèle fonctionnel comprenant une carte tridimensionnelle de ladite structure comprenant des informations fonctionnelles relatives à ladite structure mesurées en de multiples points sur ladite structure, ladite carte présentant des caractéristiques fonctionnelles de ladite structure,
ledit modèle fonctionnel comprenant un modèle électrique lorsque ladite sonde est mise en contact avec de multiples points de contact sur ladite structure, et en réponse audit capteur de position de ladite sonde, ledit processeur étant opérationnel pour obtenir des informations de position et d'orientation associées à chacun desdits points de contact et ladite structure comprenant un coeur, et lesdites informations fonctionnelles comprenant une caractéristique d'un électrocardiogramme local pris au niveau de chacun desdits points de contact ; et
un dispositif d'affichage relié audit processeur pour afficher lesdites informations fonctionnelles à partir de ladite carte en alignement avec ladite image,
**caractérisé en ce que** ledit processeur est opérationnel pour aligner ladite image avec ladite carte en identifiant automatiquement au moins l'une desdites caractéristiques fonctionnelles avec au moins une caractéristique anatomique correspondante parmi lesdites caractéristiques anatomiques dans ladite image en utilisant des valeurs de tension dans ladite carte pour identifier une desdites caractéristiques fonctionnelles avec une caractéristique anatomique correspondante parmi lesdites caractéristiques anatomiques dans l'image tridimensionnelle qui sont connues pour générer de telles valeurs.

2. Appareil selon la revendication 1, lesdites informations fonctionnelles comprenant des amplitudes de tensions électriques au niveau desdits points de contact.

3. Appareil selon la revendication 1, lesdites informations fonctionnelles comprenant des impédances entre une surface dudit corps et des points de contact respectifs desdits points de contact.

4. Appareil selon la revendication 1, ladite image étant une image tomographique calculée d'un thorax dudit corps qui comprend une représentation d'un coeur de celui-ci.

5. Appareil selon la revendication 1, ladite image étant une image ultrasonore.
